# FASCICULE DE BREVET EUROPEEN

(11) **EP 0 964 707 B1**
(45) Date de publication et mention de la délivrance du brevet: **04.10.2001**
(21) Numéro de dépôt: 98906978.6
(22) Date de dépôt: 05.02.1998
(51) Int. Cl.: A61L 27/00, C04B 35/447

(54) **PROCEDE D'ELABORATION DE SUBSTITUTS OSSEUX SYNTHETIQUES DE POROSITE CONTROLEE**
VERFAHREN ZUR HERSTELLUNG VON SYNTHETISCHEN KNOCHENERSATZMATERIALIEN MIT KONTROLLIERTER POROSITÄT
METHOD FOR PREPARING SYNTHETIC BONE SUBSTITUTES WITH CONTROLLED POROSITY

(30) Priorité: 05.02.1997 FR 9701309
(43) Date de publication de la demande: 22.12.1999
(73) Titulaire: SDGI Holdings, Inc., Wilmington, DE 19801 (US)
(72) Inventeur: RICHART, Olivier, F-59168 Boussois (FR); SZARZYNSKI, Stephan, F-59121 Haulchin (FR)
(74) Mandataire: Moncheny, Michel
(86) Numéro de dépôt international: FR9800213
(87) Numéro de publication internationale: WO9834654

(56) Documents cités:
- WO-A-92/06653
- WO-A-95/32008
- DE-A- 3 123 460
- DE-A- 4 403 509

## Description

L'invention concerne un procédé de préparation d'une céramique synthétique macro-poreuse destinée notamment à la substitution osseuse, la céramique présentant une dimension contrôlée d'interconnexion entre les pores, ainsi qu'une porosité et une taille de pores contrôlées.

Les procédés du type précité, connus de l'art antérieur, présentent un certain nombre d'inconvénients.

En effet, ces procédés ne permettent pas de maîtriser totalement l'architecture poreuse de la céramique obtenue, à savoir notamment le contrôle de la taille et de la forme des macro-pores, leur répartition au sein de la matrice céramique, mais aussi de la taille des interconnexions entre macro-pores.

Or, ce manque de contrôle diminue l'efficacité biologique des céramiques, ce qui se caractirise, dans le cas d'une application à la substitution osseuse, par une mauvaise réhabilitation osseuse ou, tout du moins, une réhabilitation partielle de celle-ci.

De plus, des hétérogénéités du comportement mécanique, notamment à la compression, sont souvent relevées du fait de la non parfaite reproductibilité des architectures.

Certains procédés préconisent d'exercer une pression sur les particules entassées, destinées à former les pores, afin de contrôler le diamètre d'interconnexion.

Cependant, ce type de procédé ne permet pas un contrôle effectif de l'interconnexion, qui soit homogène, facilement reproductible et évolutif.

Par ailleurs, les hétérogénéités des structures des substituts osseux céramiques de l'art antérieur induisent souvent des comportements mécaniques variables.

La valeur des résistances mécaniques, du fait du non contrôle total des architectures, est souvent faible, et en particulier en compression. Il est nécessaire de limiter les sollicitations mécaniques sur l'implant et par voie de conséquence, de réduire la taille des pièces fabriquées en vue de limiter les risques de faillite mécanique du système implant-os receveur.

L'état de la technique est notamment représenté par les documents WO-A-92 06653, DE-A-44 03 509, DE-A-31 23 460 et WO-A-95 32008.

L'invention a donc. pour but de pallier les inconvénients de l'art antérieur précité.

Ainsi, un objectif du procédé de l'invention est notamment de :
- contrôler l'interconnexion entre les macro-pores d'une céramique synthétique, de façon reproductible et évolutive, afin notamment de permettre le passage des cellules osseuses et d'assurer ainsi une néoformation osseuse jusqu'au coeur du biomatériau, dans le cas d'une application à la substitution osseuse ; ce contrôle de l'interconnexion devant être effectué de manière homogène et jusqu'au coeur du substitut, quelque soit sa taille ;
- maîtriser la porosité de la céramique ainsi que les dimensions des pores ;
- réaliser des céramiques biocompatibles, "à la demande", présentant des dimensions et une forme prédéfinie.

A cet effet, l'invention propose un procédé du type précité caractérisé par les étapes successives suivante :
a) construction d'un édifice d'éléments porogènes ;
b) thermo-formage de l'édifice de manière à assurer une coalescence maîtrisée entre les éléments porogènes ;
c) imprégnation de l'édifice avec une suspension de manière à combler les espaces entre les éléments porogènes ;
d) élimination des éléments porogènes de manière à générer la macro-porosité à diamètre d'interconnexion maîtrisé.

Plus précisément, suivant le procédé de l'invention des revendications 1 à 14, on empile dans un réservoir des particules d'un composé organique porogène à faible expansion thermique, les particules présentant une forme prédéterminée.

Afin de réaliser un contact étroit entre les particules, et donc de générer l'interconnexion entre macro-pores, on assure un traitement de thermo-formage sur les particules.

Cette opération a pour but d'atteindre la température de travail supérieure à la température de transition vitreuse du composé organique afin de le placer dans son plateau caoutchoutique, en vue de réaliser une soudure maîtrisée entre ces particules.

La génération du pontage maîtrisé entre particules, et donc de la future interconnexion contrôlée entre macropores, est due à la régulation du paramètre temps de traitement de thermoformage et du paramètre température de traitement de thermoformage.

D'autres approches sont possibles si l'on désire réduire le temps de traitement. Il est par exemple possible d'augmenter la température de travail (sans toutefois atteindre la température de décomposition du polymère) ou encore d'appliquer sur l'édifice polymérique (à une température supérieure à la température vitreuse) une pression afin d'accélérer le développement de la soudure qui se forme.

Une fois les connexions effectuées entre les particules, on extrait la structure monobloc formée par ces particules interconnectées, après refroidissement de la structure, pour la placer dans un moule poreux.

Puis, les espaces entre les particules sont colmatés par une poudre de phosphate de calcium en suspension en milieu aqueux, afin de constituer l'armature céramique du matériau.

Après l'élimination de l'eau par la structure poreuse du moule, le produit obtenu est démoulé puis traité thermiquement afin d'éliminer dans une première étape le composé organique, et donc de générer la porosité du produit, puis dans une seconde étape, de densifier les parois de la céramique.

Suivant l'invention, le composé organique est choisi notamment parmi les résines acryliques, telles que notamment le polyméthylmétacrylate (PMMA), le polymétacrylate (PMA), le polystyrène, le polyéthylène, ou analogue.

En outre, dans un mode de réalisation de l'invention, les particules présentent une forme générale sensiblement sphérique.

Suivant l'invention, le phosphate de calcium est choisi notamment parmi l'hydroxyapatite (HA) ou le phosphate tricalcique (TCP β), ou analogue, ou leur mélange.

Le procédé de l'invention permet ainsi d'obtenir une céramique synthétique macro-poreuse dont l'interconnexion entre macro-pores est parfaitement contrôlée et dont les pores présentent des dimensions contrôlées, et sont répartis en nombre et en surface de manière prédéterminée.

Plus précisément, le procédé de l'invention permet une maîtrise parfaite du diamètre des pores sphériques, notamment entre 100 *µ*m et 800 *µ*m avec des interconnexions parfaitement contrôlées notamment entre 0,1 et 0,8 fois le diamètre du macro-pore impliqué, et plus particulièrement entre 40 et 640 *µ*m.

Par ailleurs, la maîtrise des paramètres tels que la température, la pression et la durée de traitement permet le contrôle du diamètre d'interconnexion.

Une loi de redistribution du polymère à une température supérieure à la température de transition vitreuse, du type écoulement visqueux, pour la formation des cols entre particules, régit la coalescence des particules et autorise ainsi la maîtrise parfaite du diamètre d'interconnexion final.

Pour des raisons expérimentales de mise en oeuvre, on réalise des céramiques dont le diamètre d'interconnexion entre les macro-pores est supérieur à 30 *µ*m. En conséquence, pour chaque classe granulométrique donnée de billes, il est possible d'ajuster précisément la coalescence et donc l'interconnexion finale du produit. Il est entendu que ces deux paramètres sont distincts l'un de l'autre et peuvent être ajustés de façon indépendante.

L'invention permet également d'obtenir des céramiques du type précité de toutes les tailles, qu'elles soient faibles ou importantes (plusieurs cm³) et présentant une résistance importante aux sollicitations mécaniques.

La céramique synthétique peut présenter une structure à gradients de porosité, comportant on non des parties denses.

En outre, ce procédé permet également d'obtenir des céramiques, pouvant être des substituts osseux, de forme complexe et de porosité constante ou non.

Enfin, selon un autre aspect, l'invention concerne une utilisation d'une telle céramique en tant que substitut osseux.

D'autres caractéristiques et avantages de l'invention ressortiront de la description qui suit en référence aux dessins et représentations annexés.

La figure 1 représente une vue en microscopie électronique montrant la coalescence de particules de polymère.

La figure 2 représente une vue en microscopie électronique d'une structure monobloc formée par les particules interconnectées.

Les figures 3 et 4 représentent des vues en microscopie électronique de céramiques comportant des pores de 500 *µ*m et dont les diamètres d'interconnexion sont respectivement de 100*µ*m et 200 *µ*m.

La figure 5 représente une vue en microscopie électronique de particules ayant subi un traitement thermique à 150°C.

Les figures 6a à 6c sont des vues schématiques en perspective de modes de réalisation de substituts osseux à renforcement mécanique périphérique.

Les figures 7a et 7b sont des vues schématiques en perspective de modes de réalisation de substituts osseux à renforcement mécanique tubulaire.

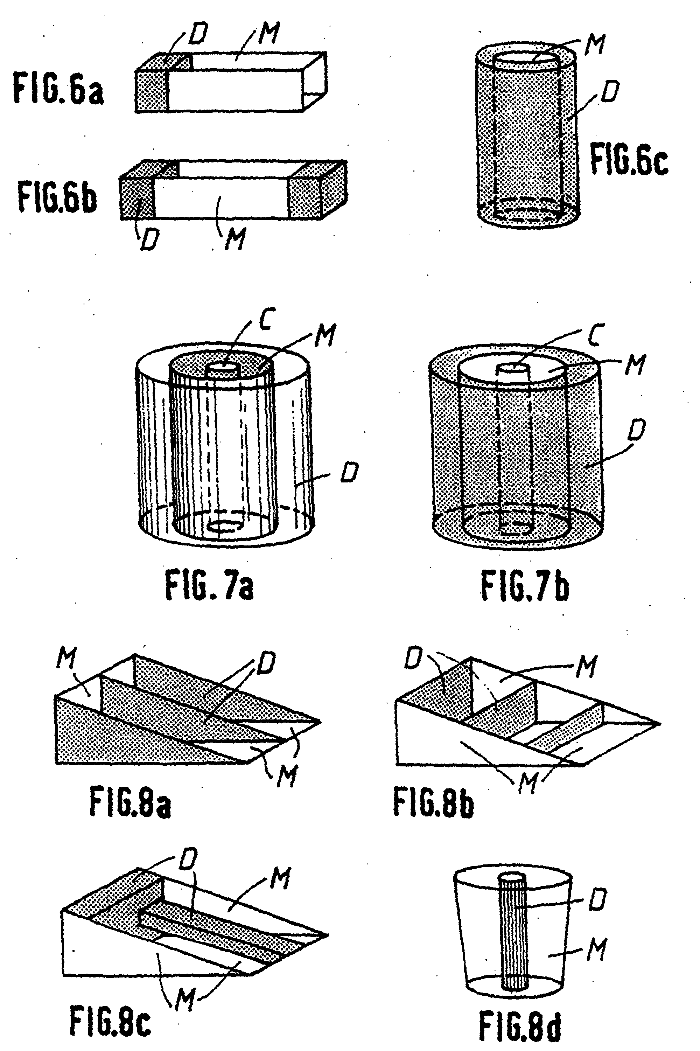

Les figures Sa à 8d sont des vues schématiques en perspective de substituts osseux montrant des applications possibles, notamment pour une ostéotomie tibiale d'addition (figures 8a à 8c).

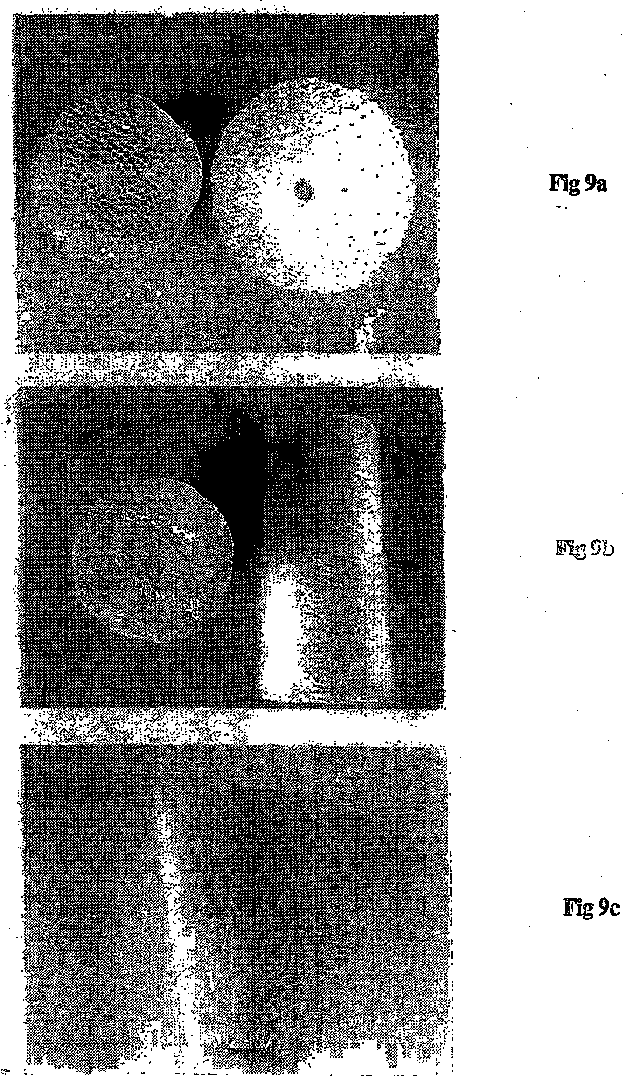

Les figures 9a à 9c sont des micrographies optiques représentant le substitut de la figure 8d, vu respectivement de dessus, côté et en coupe longitudinale.

Dans un mode de réalisation particulier, la céramique macro-poreuse est préparée à partir de particules de résines acryliques, telles que des particules de polyméthylmétacrylate (PMMA), empilées dans un réservoir.

On peut également envisager d'utiliser des particules de polyéthylène, de polymétacrylate ou de polystyrène.

Le point commun entre ces composés est notamment leur caractéristique de porogène.

D'une manière générale, on peut choisir tout composé organique présentant une faible expansion thermique pour éviter la détérioration du composé lors d'un cycle thermique.

On choisira par conséquent un polymère thermoplastique, apte au thermo-formage.

Par ailleurs, dans un mode de réalisation particulier, on utilisera un polymère de structure au moins partiellement amorphe, de préférence totalement amorphe, afin d'éviter une augmentation de volume trop importante lors du traitement thermique.

Enfin, l'élément porogène doit pouvoir se dégrader à basse température avec un taux d'impuretés résiduelles négligeable et des produits de décomposition non corrosifs.

Typiquement, le PMMA correspond à un composé présentant l'ensemble de ces caractéristiques.

Ainsi, dans la suite de la description, on fera référence à des particules de PMMA, étant entendu que tout autre composé ayant les caractéristiques précitées peut être utilisé.

Les particules de PMMA utilisées se présentent sous la forme de billes pouvant présenter des dimensions sensiblement identiques entre elles ou des dimensions différentes.

Dans ce dernier cas, on peut envisager de placer dans le fond du réservoir un certain nombre de billes de petite taille sur lesquelles reposent des billes de plus grosse taille. Une telle disposition permettra d'obtenir une céramique présentant des porosités différentes.

On peut également envisager de disposer dans le réservoir différentes couches de billes, le diamètre des billes étant croissant au fur et à mesure de leur empilement. On obtient ainsi une céramique à gradient de porosité.

La forme sphérique des particules de PMMA permet notamment d'assurer des contacts étroits et divers entre les particules, d'obtenir une porosité de morphologie homogène et de maîtriser le volume poreux final de la céramique.

En effet, les particules de PMMA ont la particularité d'être peu ou pas déformables, comme indiqué précédemment.

De ce fait, les pores du substitut obtenu présentent des dimensions sensiblement identiques à celles des particules.

Les billes de PMMA utilisées présentent, dans un mode de réalisation particulier, une granulométrie dont la distribution s'étend de quelques microns à 850 microns environ.

Afin de contrôler les dimensions des macro-pores de la céramique obtenue, la poudre de PMMA subit au préalable un tamisage mécanique.

A cet effet, la poudre de PMMA est passée entre des tamis d'ouverture de mailles différentes, de manière à obtenir des lots de poudre de classes granulométriques relativement étroites.

On peut ainsi sélectionner des classes granulomëtriques allant de 0 à 100 *µ*m, de 100 à 200 *µ*m, de 200 à 300 *µ*m, de 400 à 500 *µ*m, de 500 à 600 *µ*m, de 600 à 700 *µ*m, ou de 700 à 850 *µ* m.

Il est entendu que des classes granulométriques plus étroites -par exemple de 190 à 200 *µ*m- peuvent être sélectionnées.

Il est à noter que certaines des billes de PMMA peuvent être creuses.

Il est entendu que le nombre, la forme et la répartition des particules utilisées est fonction de la forme du moule et de la céramique que l'on souhaite obtenir.

Le réservoir destiné à recevoir les particules de composé organique doit pouvoir résister au moins à la température de dégradation thermique dudit composé.

Dans le cas présent où une résine acrylique, et plus particulièrement le PMMA, est utilisée, cette température est de l'ordre de 200°C.

Ainsi, le réservoir est par exemple métallique, céramique, ou polymérique.

Dans une seconde étape, on assure un traitement de thermo-formage sur les particules de PMMA afin d'assurer une coalescence entre lesdites particules.

En effet, un polymère amorphe, tel que notamment le PMMA, prend une consistance caoutchoutique à une température supérieure à la température de transition vitreuse T_{V} dudit polymère.

On rappellera que la température de transition vitreuse du PMMA est de l'ordre de 110°C.

Aussi, à une température supérieure à la température de transition vitreuse, le polymère subit des modifications de ses macro-molécules pour arriver à la consistance caoutchoutique proche de l'état visqueux. Le polymère est alors facilement modelable.

Dans cet état de plasticité plus ou moins important de la matière, un contact des particules les unes avec les autres permet, par des mécanismes de diffusion, un enchevêtrement au moins partiel de certaines des macro-molécules contenues dans les différentes particules, aboutissant au soudage de celles-ci.

Une fois que l'état souhaité est obtenu, il est fixé par simple retour à température ambiante.

En effet, la viscosité du matériau obtenu augmente ensuite progressivement lorsque la température décroît.

Le procédé de thermo-formage consiste, d'une manière générale, à chauffer, de façon homogène, les particules, prises dans leur ensemble ou à leur surface, à une température supérieure à la température de transition vitreuse du composé organique.

Le traitement de thermo-formage du PMMA peut se décomposer selon les étapes suivantes :
- préchauffage du réservoir à vide, à une température supérieure à celle de la transition vitreuse du PMMA, en l'occurrence supérieure à environ 110°C ;
- introduction des billes de PMMA dans le réservoir ;
- formage et soudage des billes jusqu'à la fin du refroidissement ;
- refroidissement à la température ambiante.

Suivant un autre mode de réalisation, on peut prévoir de ne pas préchauffer le réservoir à vide mais de le chauffer une fois rempli.

La technique de thermo-formage de ces éléments porogènes est reproductible et également applicable à des édifices de grande surface (plusieurs dizaines de centimètres carré et de gros volume (plusieurs dizaines de centimètres cubes).

L'efficacité du processus pour une maîtrise du diamètre d'interconnexion est essentiellement contrôlée par la diffusion, la qualité de la soudure va dépendre de la température, du temps et de l'intensité du contact entre particules.

Afin de déterminer la température optimale de façonnage du polymère, les billes de PMMA sont introduites dans un réservoir métallique préalablement chauffé par exemple aux différentes températures de traitement.

Ces températures, supérieures à la température de transition vitreuse, sont égales respectivement à 120°C, 150°C, 180°C, et 200°C.

Ces essais ont permis de suivre l'évolution du pontage entre billes en fonction de la durée de thermoformage. Cette analyse est effectuée par microscopie électronique à balayage.

Ces essais sont effectués, dans un premier temps, sur des billes de PMMA de distribution granulométrique comprise entre 500 et 600 *µ*m. Pour chaque manipulation, un poids constant de billes égal à 5 grammes est introduit dans un réservoir cylindrique de diamètre de 26 millimètres.

Pour une température de traitement égale à 120°C et des durées de chauffage supérieures à 20 heures, les billes ne manifestent aucune cohésion entre elles. Cette température parait insuffisante pour permettre dans les délais considérés une diffusion et un pontage suffisant entre les billes de polymère.

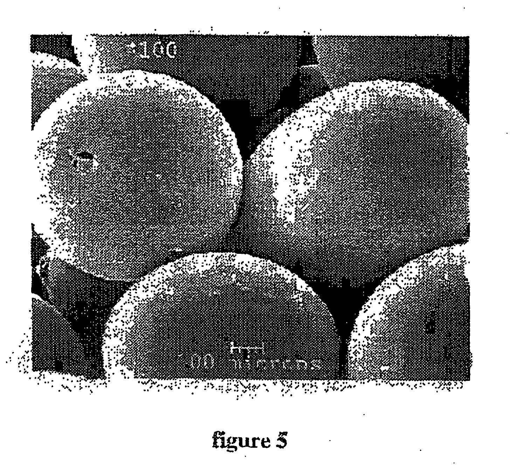

Cette cohésion entre particules devient apparente à une température de 150°C (voir la figure 5 où les particules de diamètre compris entre 500 et 600 *µ*m ont subi un traitement à 150°C pendant 16 heures).

Cependant, des temps importants sont nécessaires afin d'établir une soudure correcte entre les particules de PMMA. L'analyse par microscopie électronique à balayage d'échantillons traités 16 heures permet de visualiser l'empreinte entre les billes et donc d'évaluer l'efficacité de l'opération de thermo-formage.

Cette empreinte correspond à la future interconnexion entre les macropores de la biocéramique. Dans le cas présent, cette trace demeure discrète et d'un diamètre estimé à 100 *µ*m.

Une température d'essai de 180°C permet, pour de faibles durées de traitement, un pontage significatif entre les billes.

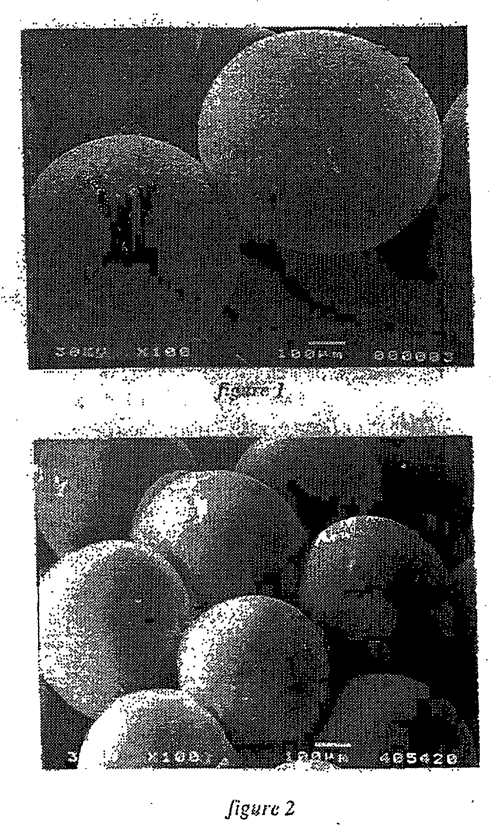

Les particules de PMMA thermo-formées forment ainsi une structure monobloc comportant un certain nombre d'espaces entre les billes (voir figures 1 et 2).

On extrait ensuite cette structure monobloc du réservoir pour la placer dans un moule poreux.

ce moule est, dans le présent mode de réalisation, en plâtre.

on peut également envisager d'utiliser un moule en céramique, en métal ou en résine, ou analogue.

Les espaces entre billes sont alors remplis, imprégnés par une suspension dense à base de phosphate de calcium en milieu aqueux.

Cette suspension, ou barbotine, comporte de l'hydroxyapatite (HA) ou du phosphate tricalcique (TCP β), ou bien leur mélange jusqu'à hauteur de 100 % chacun.

La suspension, ainsi constituée de poudre et d'eau, va peu à peu sécher autour de la structure monobloc grâce à des phénomènes de depression capillaire générés par la porosité du plâtre.

Un produit "cru", mélange biphasé de céramique et de billes de polymère, est alors obtenu.

Dans un mode de réalisation particulier de l'invention, le moule comporte, dans ses parois, une réserve contenant une quantité suffisante de barbotine.

Cette réserve permet d'imprégner en continu la structure monobloc de manière que tous les espaces soient parfaitement comblés, et ce, jusqu'au sèchage de la barbotine. Cette réserve est utilisée en combinaison avec le coulage de la barbotine.

Lors du coulage de la suspension, une étape de défloculation est réalisée pour parvenir à une désagglomération optimale de la suspension.

La défloculation d'une suspension de phosphate de calcium, par exemple d'hydroxyapatite, est assurée par un polyélectrolyte de type carboxylate, largement utilisé en céramurgie : le polyacrylate d'ammonium (PaA).

A partir de conditions déterminées (0,6% de PaA, pH 11), différentes suspensions ont été testées avec des teneurs en HA croissantes, soit 82%, 84% et 86%.

Les viscosités de ces barbotines sont ensuite déterminées à un gradient de vitesse de 100 s⁻¹.

Les résultats montrent qu'une suspension à 82% de matière sèche possède une viscosité relativement faible qui permet un remplissage total des interstices présents entre les sphères de PMMA.

Il est entendu que des suspensions moins concentrées en taux de matière sèche peuvent être utilisées notamment pour diminuer la viscosité et faciliter l'imprégnation de la structure polymérique.

Le produit "cru" obtenu est ensuite démoulé.

Puis, afin de générer une porosité, le produit "cru" subit un traitement thermique à basse température, mais supérieure à la température de dégradation thermique du composé utilisé (environ 200°C dans le cas présent).

Dans le présent mode de réalisation, la température de ce traitement est inférieure à 300°C environ.

Ce traitement thermique va donc permettre d'éliminer les billes en brûlant toutes les matières organiques et donc de générer des vides à leur emplacement.

Enfin, pour augmenter la cohésion et la rigidité du produit, celui-ci subit un traitement thermique de frittage à une température comprise entre 1.100°C et 1.300°C.

Une céramique macro-poreuse aux interconnexions maîtrisées est ainsi obtenue et peut être destinée à la substitution osseuse.

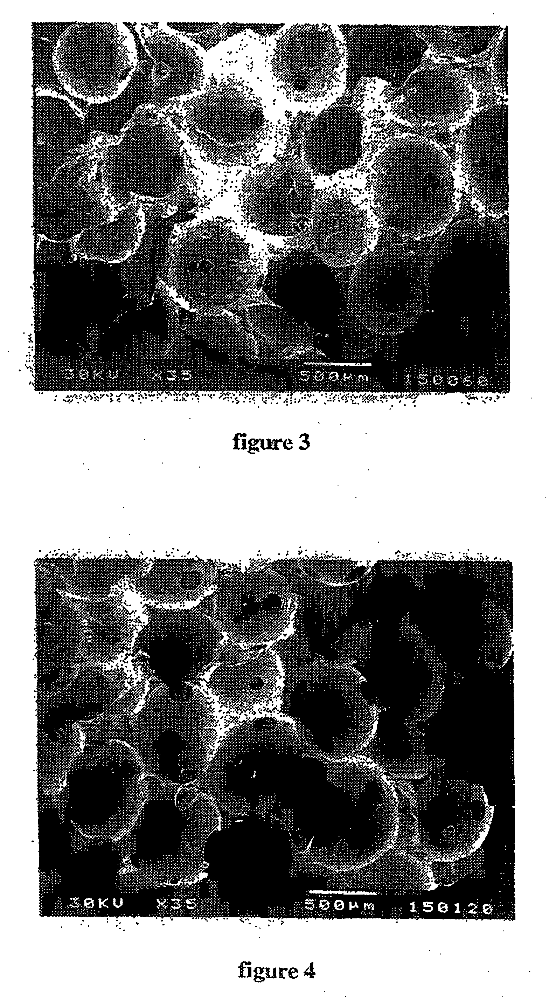

Les figures 3 et 4 illustrent bien la maîtrise et la croissance du diamètre d'interconnexion (petits trous noirs) pour une porosité de 500 *µ*m.

Il est entendu que le moule en plâtre peut présenter différentes formes, qu'elles soient simples ou complexes.

Ainsi, il peut présenter par exemple une forme sensiblement parallélépipédique ou cylindrique, s'agissant des formes simples.

En outre, on peut prévoir de ménager à l'intérieur d'un tel moule des parois de formes diverses, de manière à obtenir une céramique de forme complexe.

Il est en effet entendu que la forme du moule en plâtre détermine celle de la céramique a réaliser.

Les figures 6a à 9c montrent des exemples de configuration complexe de la céramique obtenue, et donc du moule.

On peut ainsi prévoir des parties denses permettant un renforcement mécanique de certaines zones du substitut.

L'édifice polymérique ne comporte pas alors de billes à l'endroit où la (les) partie(s) dense(s) est (sont) souhaitée(s).

Sur les figures 6a à 6c, les parties D les plus denses sont situées à la périphérie du substitut, qu'il soit parallélépipèdique (figures 6a et 6b) ou cylindrique (figure 6c).

On peut également envisager des parties denses insérées entre des parties macro-poreuses (non représenté).

En outre, les figures 7a et 7b montrent un mode de réalisation de substitut cylindrique comportant une partie dense D, une partie macro-poreuse M et une partie creuse C.

Sur la figure 7a, la partie dense D est insérée entre la partie creuse C et la partie macro-poreuse M, tandis que sur la figure 7b, la partie dense est située à la périphérie du substitut.

Enfin, les figures 8a à 8c montrent des substituts de forme complexe, dits en forme de "coin", où les parties denses, ou micro-poreuses, sont représentées en D et les parties macro-poreuses en M.

Dans ces modes de réalisation, les parties M sont intercalées entre les parties D.

De tels substituts peuvent par exemple être utilisés dans des cas d'ostéotomie tibiale d'addition.

La figure 8d, ainsi que les figures 9a à 9c, représentent un substitut de forme générale tronconique comportant une partie dense D interne de forme générale cylindrique et une partie poreuse externe sensiblement tronconique.

Les parties denses D sont obtenues par exemple en générant une porosité plus faible, par exemple, en utilisant des particules de tailles inférieures.

La résistance du substitut -et donc sa densité- peut être également renforcée en augmentant les espaces entre les particules, de manière à augmenter la quantité de barbotine imprégnée, ou en supprimant les particules dans certaines zones et en remplissant ces zones de barbotine.

## Revendications

1. Procédé de préparation d'une céramique synthétique macro-poreuse destinée notamment à la substitution osseuse, présentant une porosité, une taille de pores et une dimension d'interconnexion contrôlées, **caractérisé en ce qu'**il comprend successivement les étapes suivantes :
a)on empile, dans un réservoir, des particules d'un composé organique porogène, les particules ayant une forme prédéterminée ;
b)on assure un traitement de thermo-formage sur lesdites particules, pour assurer une coalescence entre lesdites particules, à une température supérieure à celle de la transition vitreuse du composé organique ;
c) on extrait les particules thermo-formées du réservoir et on les place dans un moule poreux ;
d)on coule une suspension à base de phosphate de calcium, ou analogue, en milieu aqueux au travers des particules, la suspension remplissant les espaces entre lesdites particules ;
e) une fois que le moule poreux a absorbé l'eau en excédent, on démoule le produit obtenu ;
f) on assure un traitement thermique de déliantage, pour brûler toutes les matières organiques, et générer la porosité du produit ;
g) on chauffe le produit à une température suffisante pour le frittage et donc la consolidation du produit.

2. Procédé selon la revendication 1, **caractérisé en ce que** l'étape b) de thermo-formage consiste à chauffer, de façon homogène, les éléments porogènes, pris dans leur ensemble ou à leur surface, à une température supérieure à la température de transition vitreuse du composé organique.

3. Procédé selon la revendication 1 ou 2, **caractérisé en ce que** l'étape b) de thermo-formage comporte les étapes suivantes :
- préchauffage du réservoir à vide, à une température supérieure à celle de la transition vitreuse du composé organique ;
- introduction des particules de composé organique dans le réservoir ;
- formage et soudage des particules jusqu'à la fin du refroidissement ;
- refroidissement à la température ambiante.

4. Procédé selon l'une quelconque des revendications 2 et 3, **caractérisé en ce que** le composé organique est un polymère thermoplastique apte au thermo-formage.

5. Procédé selon la revendication 4, **caractérisé en ce que** le composé organique est de structure au moins partiellement amorphe, de préférence amorphe.

6. Procédé selon l'une quelconque des revendications 1 à 5, **caractérisé en ce que** le composé organique est choisi notamment parmi les résines acryliques, telles que le polyméthylmátacrylate (PMMA), le polymétracrylate (PMA), le polystyrène, le polyéthylène, ou analogue.

7. Procédé selon l'une des revendications 1 à 6, **caractérisé en ce que** l'étape a) est réalisée à l'aide de particules présentant une forme générale sensiblement sphérique.

8. Procédé selon l'une quelconque des revendications 1 à 7, **caractérisé en ce que** le phosphate de calcium est choisi notamment parmi l'hydroxyapatite (HA) ou le phosphate tricalcique (TCP β), ou analogue, ou leur mélange.

9. Procédé selon l'une quelconque des revendications 1 à 8, **caractérisé en ce que** le traitement thermique de l'étape f) est effectué à une température supérieure ou égale à la température de dégradation thermique du composé organique.

10. Procédé selon l'une quelconque des revendications 1 à 9, **caractérisé en ce que** le traitement thermique de l'étape g) est réalisé à une température de l'ordre de 1.000°C à 1.300°C.

11. Procédé selon l'une quelconque des revendications 1 à 10, **caractérisé en ce que** le réservoir est métallique, céramique, polymérique ou en tout autre matériau résistant au moins à la température de dégradation thermique du composé organique utilisé.

12. Procédé selon l'une quelconque des revendications 1 à 11, **caractérisé en ce que** le moule poreux présente une forme particulière prédéterminée correspondant à celle de la céramique à réaliser.

13. Procédé selon l'une quelconque des revendications 1 à 12, **caractérisé en ce que** le moule poreux est choisi notamment parmi les moules en plâtre, céramique, métal ou résine.

14. Procédé selon l'une des revendications 1 à 13, **caractérisé en ce que** ladite céramique présente un diamètre de pores compris entre 100 et 800µm avec des interconnexions entre 0,1 et 0,8 fois le diamètre du macropore impliqué.

15. Céramique synthétique obtenue par la mise en oeuvre du procédé selon l'une des revendications 1 à 14, **caractérisée en ce que** le diamètre des pores est compris entre 100 et 800µm avec des interconnexions entre 0,1 et 0,8 fois le diamètre du macropore impliqué.

16. Céramique synthétique selon la revendication 15, **caractérisée en ce qu'**elle présente une structure à gradients de porosité, comportant ou non des parties denses.

## Claims

1. A method for preparing a macro-porous synthetic ceramic intended in particular as bone substitute, with controlled porosity, pore size and interconnection dimension, **characterised by** the fact that it comprises in turn the following stages:
a) particles of a porogenous organic compound are packed into a vessel, the particles being of a predetermined shape;
b) thermoforming treatment is given to the said particles to ensure coalescence between the said particles at a temperature higher than that of the vitreous transition of the organic compound;
c) the thermoformed particles in the vessel are extracted and put into a porous mould;
d) a calcium phosphate based or similar suspension is poured in an aqueous milieu through the particles, the suspension filling the spaces between the said particles;
e) once the porous mould has absorbed the surplus water, the resultant product is removed from the mould;
f) a debonding heat treatment is given to bum off all the organic materials and create porosity in the product;
g) the product is heated to a sufficient temperature for sintering and therefore consolidating the product.

2. A method according to Claim 1, **characterised by** the fact that the thermoforming stage b) consists in uniformly heating the porogenous parts, taken in their entirety or at their surface, to a temperature higher than the vitreous transition temperature of the organic compound.

3. A method according to Claim 1 or 2, **characterised by** the fact that the thermoforming stage b) covers the following stages:
- preheating of the empty vessel to a temperature higher than that of the vitreous transition of the organic compound;
- putting the organic compound particles into the vessel;
- forming and welding the particles up to the end of the cooling;
- cooling at ambient temperature.

4. A method according to any one of the Claims 2 and 3, **characterised by** the fact that the organic compound is a thermoplastic polymer suitable for thermoforming.

5. A method according to Claim 4, **characterised by** the fact that the organic compound has an amorphous structure at least partly, and preferably an amorphous structure.

6. A method according to any one of the Claims 1 to 6, **characterised by** the fact that the organic compound is chosen particularly from among the acrylic resins such as polymethyl methacrylate (PMMA), polymethacrylate (PMA), polystyrene, polyethylene or the like.

7. A method according to one of the Claims 1 to 6, **characterised by** the fact that stage a) is performed with particles which are more or less spherical in shape.

8. A method according to any one of the Claims 1 to 7, **characterised by** the fact that the calcium phosphate is chosen in particular from among hydroxy apatite (HA) or tricalcium phosphate (TCP 8) or the like, or a mixture of them.

9. A method according to any one of the Claims 1 to 8, **characterised by** the fact that the heat treatment in stage f) is done at a temperature above or equal to the temperature of thermal degradation of the organic compound.

10. A method according to any one of the Claims 1 to 9, **characterised by** the fact that the heat treatment in stage g) is done at a temperature in the region of 1000°C to 1300°C.

11. A method according to any one of the Claims 1 to 10, **characterised by** the fact that the vessel is metal, ceramic, polymeric or any other material resistant at least to the thermal degradation temperature of the organic compound used.

12. A method according to any one of the Claims 1 to 11, **characterised by** the fact that the porous mould has a particular predetermined shape corresponding to that of the ceramic to be made.

13. A method according to Claims 1 to 12, **characterised by** the fact that the porous mould is chosen in particular from among plaster, ceramic, metal or resin moulds.

14. A method according to one of the Claims 1 to 13, **characterised by** the fact that the said ceramic has a pore diameter between 100 and 800 µm with interconnections between 0.1 and 0.8 times the diameter of the macropore involved.

15. Synthetic ceramic obtained by using the method according to one of the Claims 1 to 14, **characterised by** the fact that the diameter of the pores is between 100 and 800 µm with interconnections between 0.1 and 0.8 times the diameter of the macropore involved.

16. Synthetic ceramic according to Claim 15, **characterised by** the fact that it has a porosity gradient structure which has or does not have dense sections.

## Patentansprüche

1. Verfahren zur Herstellung einer makroporösen synthetischen Keramik, die insbesondere zur Knochensubstitution bestimmt ist und eine kontrollierte Porosität, Porengröße und Abmessung der Verbindungen besitzt, **dadurch gekennzeichnet, dass** es nacheinander die folgenden Schritte umfasst:
a) Einfüllen von Teilchen einer porogenen organischen Verbindung in einen Behälter, wobei die Teilchen eine vorbestimmte Form haben;
b) Unterwerfen der Teilchen einer Wärmeumformungsbehandlung, damit eine Verbindung zwischen diesen Teilchen gewährleistet wird, bei einer Temperatur über der Glasübergangstemperatur der organischen Verbindung;
c) Entnehmen der wärmeumgeformten Teilchen aus dem Behälter und Einbringen in eine poröse Form;
d) Gießen einer Suspension auf der Basis von Calciumphosphat oder einem Analogon in wässrigem Medium über die Teilchen, wobei die Suspension die Räume zwischen den Teilchen ausfüllt;
e) Entnehmen des erhaltenen Produkts aus der Gußform, sobald die poröse Form das überschüssige Wasser absorbiert hat;
f) Durchführen einer thermischen Ablösungsbehandlung, damit die gesamten organischen Substanzen verbrannt werden und die Porositat des Produkts erzeugt wird;
g) Erhitzen des Produkts bei einer ausreichenden Temperatur für das Sintern und somit die Verfestigung des Produkts.

2. Verfahren nach Anspruch 1, **dadurch gekennzeichnet, dass** der Wärmeumformungsschritt b) das homogene Erhitzen der porogenen Bestandteile in ihrer Gesamtheit oder an ihrer Oberfläche bei einer Temperatur über der Glasübergangstemperatur der organischen Verbindung umfasst.

3. Verfahren nach Anspruch 1 oder 2, **dadurch gekennzeichnet, dass** der Wärmeumformungsschritt b) die folgenden Schritte umfasst;
- vorheizen des leeren Behälters bei einer Temperatur über der Glasübergangstemperatur der organischen Verbindung;
- Einbringen der Teilchen der organischen Verbindung in den Behälter;
- Formen und Verschweißen der Teilchen bis zum Ende des Abkühlens;
- Abkühlen bei Umgebungstemperatur.

4. Verfahren nach einem der Ansprüche 2 und 3, **dadurch gekennzeichnet, dass** die organische Verbindung ein zur Wärmeumformung geeignetes Thermoplastpolymer ist.

5. Verfahren nach Anspruch 4, **dadurch gekennzeichnet, dass** die organische Verbindung zumindest teilweise eine amorphe Struktur besitzt und vorzugsweise amorph ist.

6. Verfahren nach einem der Ansprüche 1 bis 5, **dadurch gekennzeichnet, dass** die organische Verbindung insbesondere ausgewählt ist aus Acrylharzen, wie Polymethylmethacrylat (PMMA), Polymethacrylat (PMA), Polystyrol, Polyethylen, oder einem Analogon.

7. Verfahren nach einem der Ansprüche 1 bis 6, **dadurch gekennzeichnet, dass** der Schritt a) mit Hilfe von Teilchen durchgeführt wird, die eine deutlich kugelförmige allgemeine Form haben.

8. Verfahren nach einem der Ansprüche 1 bis 7, **dadurch gekennzeichnet, dass** das Calciumphosphat insbesondere ausgewählt ist aus Hydroxylapatit (HA) oder Tricalciumphosphat (TCP β) oder einem Analogon oder einem Gemisch davon.

9. Verfahren nach einem der Ansprüche 1 bis 8, **dadurch gekennzeichnet, dass** die thermische Behandlung von Schritt f) bei einer Temperatur über oder gleich der thermischen Zersetzungstemperatur der organischen Verbindung ist.

10. Verfahren nach einem der Ansprüche 1 bis 9, **dadurch gekennzeichnet, dass** die thermische Behandlung von Schritt g) bei einer Temperatur in der Größenordnung von 1000°C bis 1300°C durchgeführt wird.

11. Verfahren nach einem der Ansprüche 1 bis 10, **dadurch gekennzeichnet, dass** der Sehalter metallisch, keramisch, aus einem Polymer oder allen anderen Materialien ist, die zumindest bis zur thermischen Zersetzungstemperatur der verwendeten organischen Verbindung beständig sind.

12. Verfahren nach einem der Ansprüche 1 bis 11, **dadurch gekennzeichnet, dass** die poröse Gußform eine besondere vorbestimmte Form besitzt, die derjenigen der herzustellenden Keramik entspricht.

13. Verfahren nach einem der Ansprüche 1 bis 12, **dadurch gekennzeichnet, dass** die poröse Gußform insbesondere ausgewählt ist aus Gips-, Keramik-, Metall- oder Harzformen.

14. Verfahren nach einem der Ansprüche 1 bis 13, **dadurch gekennzeichnet, dass** die Keramik einen Porendurchmesser von 100 bis 800 µm besitzt, wobei die Verbindungen das 0,1- bis 0,8fache des Durchmessers der vorhandenen Makropore umfassen.

15. Synthetische Keramik, die durch das Verfahren nach einem der Ansprüche 1 bis 14 erhalten wird, **dadurch gekennzeichnet, dass** der Durchmesser der Poren 100 bis 800 um ist, wobei die Verbindungen das 0,1- bis 0,8 fache des Durchmessers der vorhandenen Makropore umfassen.

16. Synthetische Keramik nach Anspruch 15, **dadurch gekennzeichnet, dass** ihre Struktur Porositätsgradienten aufweist, wobei dichte Abschnitte vorhanden sind oder fehlen.
